Europäisches Patentamt

(19) European Patent Office

Office européen des brevets

(11) Veröffentlichungsnummer: **0 127 092**
**B1**

(12) **EUROPÄISCHE PATENTSCHRIFT**

(45) Veröffentlichungstag der Patentschrift:
22.04.87

(51) Int. Cl.⁴: **F 01 K 23/06**, F 02 C 3/28, C 07 C 29/15

(21) Anmeldenummer: 84105696.3

(22) Anmeldetag: 18.05.84

(54) **Mittellastkraftwerk mit einer integrierten Kohlevergasungsanlage.**

(30) Priorität: 31.05.83 DE 3319732
29.07.83 DE 3327367

(43) Veröffentlichungstag der Anmeldung:
05.12.84 Patentblatt 84/49

(45) Bekanntmachung des Hinweises auf die Patenterteilung:
22.04.87 Patentblatt 87/17

(84) Benannte Vertragsstaaten:
AT BE CH DE FR GB IT LI LU NL SE

(56) Entgegenhaltungen:
DE - A - 2 024 301
DE - A - 2 425 939
FR - A - 877 792
GB - A - 189 789
GB - A - 2 075 124
US - A - 3 849 662
US - A - 3 872 025
US - A - 4 158 145
US - A - 4 277 416
US - A - 4 282 187

(73) Patentinhaber: KRAFTWERK UNION
AKTIENGESELLSCHAFT, Wiesenstrasse 35,
D-4330 Mülheim (Ruhr) (DE)

(72) Erfinder: Goebel, Konrad, Dipl.-Ing., Stettiner Strasse 5,
D-8520 Erlangen (DE)
Erfinder: Müller, Rainer, Dr. Dipl.-Ing., Herdegenplatz 1,
D-8520 Erlangen (DE)
Erfinder: Schiffers, Ulrich, Dr. Dipl.-Ing.,
Moritzbergstrasse 1, D-8501 Eckental (DE)

(74) Vertreter: Mehl, Ernst, Dipl.-Ing. et al, Postfach 22 01 76,
D-8000 München 22 (DE)

## Beschreibung

Die Erfindung bezieht sich auf ein Mittellastkraftwerk mit einer integrierten Kohlevergasungsanlage, mit einem an die Kohlevergasungsanlage angeschlossenen Gasturbinenkraftwerksteil, mit einem an die Rohgas-Wärmetauscheranlage der Kohlevergasungsanlage angeschlossenen Dampfkraftwerksteil, mit einer aus mehreren untereinander parallel geschalteten Modulen bestehenden Methanolsyntheseanlage und mit einem die Methanolsyntheseanlage mit dem Gasturbinenkraftwerksteil verbindenden Reingasverteilungssystem, welches eine Reingas-Durchströmzwischenspeicheranlage umfasst und gasseitig der Rohgas-Wärmetauscheranlage nachgeschaltet ist.

Die Druckschrift EP-A-0 127 093, veröffentlicht ein Mittellastkraftwerk zur Erzeugung von Strom und Methanol zum Gegenstand, bei dem ein kombiniertes Gasturbinen-Dampfkraftwerk und eine aus mehreren separat zuschaltbaren Modulen aufgebaute Methanolsyntheseanlage über ein Reingasverteilungssystem an einer Kohlevergasungsanlage angeschlossen ist. Die Abwärme des Rohgases wird dem Dampfkraftwerksteil über eine Rohgas-Wärmetauscheranlage nutzbar zugeführt. Bei diesem Mittellastkraftwerk lässt sich die erzeugte elektrische Leistung rasch an den jeweiligen Leistungsbedarf des elektrischen Netzes anpassen, ohne dass ein weiterer teuerer Zweitbrennstoff bei Lastspitzen eingesetzt werden muss und ohne dass bei plötzlicher Lastverminderung oder gar bei störungsbedingtem Lastabwurf ein Brennstoffverlust in Kauf genommen werden muss. Stattdessen wird bei diesem Mittellastkraftwerk in Zeiten verminderten Bedarfs an elektrischer Leistung verstärkt Methanol erzeugt und werden Überschüsse wie Mindermengen an Reingas durch die dem Reingastverteilungssystem zugeordnete Reingas-Durchström-Zwischenspeicheranlage aufgefangen.

Daher kann die vergleichsweise trägere Kohlevergasungsanlage unabhängig von den jeweiligen Lastanforderungen des elektrischen Netzes mit konstanter Leistung weitergefahren werden. Weil das der Methanolsyntheseanlage zuströmende Reingas in seiner Zusammensetzung weit von dem für die Methanolsynthese erforderlichen stöchiometrischem Verhältnis entfernt ist, muss in Zeiten verminderten Energiebedarfs, wenn das nicht vollständig umgesetzte Synthesegas nicht mehr in der Brennkammer der Gasturbine verbrannt werden kann, das in den Methanolsynthesereaktoren der einzelnen Module zurückgeführte Synthesegas mit Wasserstoff angereichert werden. Diese Wasserstoffanreicherung könnte durch externe Einspeisung von Wasserstoff erreicht werden.

Der Erfindung liegt die Aufgabe zugrunde, einen Weg zu weisen, wie bei einem Mittellastkraftwerk der eingangs genannten Art der für die Wasserstoffanreicherung des Synthesegases der Methanolsyntheseanlage erforderliche Wasserstoff im Kraftwerk selbst in möglichst wirtschaftlicher Weise gewonnen werden kann.

Bei einem Mittellastkraftwerk der eingangs genannten Art ist daher erfindungsgemäss der Methanolsyntheseanlage zur Wasserstoffanreicherung ein an die Rohgaswärmetauscheranlage angeschlossener, aus Sättiger, Konvertierungsanlage, Kühler und nachgeschalteter Gasreinigungsanlage bestehender sogenannter «Kühler-Sättiger-Kreislauf» zugeordnet in welchem Sättiger Heisswasser aus einem Wärmetauscher der Rohgas-Wärmetauscheranlage eingespeist wird und welches Wasser darin bis zur Sättigung des Gases verdampft. In einem solchen Kühler-Sättiger-Kreislauf kann durch Anreicherung des Synthesegases mit Wasserdampf und anschliessender Konvertierung des Synthesegas-Wasserdampfgemisches Wasserstoff und Kohlendioxyd erzeugt werden. Nach Abtrennen des Kohlendioxyds lässt sich das verbleibende mit Wasserstoff angereicherte Synthesegas wieder in die Methanolsyntheseanlage zurückleiten.

Alternativ lässt sich bei einem Mittellastkraftwerk der eingangs genannten Art der Methanolsyntheseanlage erfindungsgemäss eine Wasserelektrolyseanlage zuordnen, deren Wasserstoffleitung mit der Methanolsyntheseanlage verbunden und deren Sauerstoffleitung an den Kohlevergaser angeschlossen ist und die über die Generatoren mit im wesentlichen der Menge an elektrischer Energie versorgt werden kann die der Differenz zwischen Nennleistung und momentaner Last entspricht. Bei einer solchen Anlage kann der in Zeiten verminderten Strombedarfs überschüssig erzeugte elektrische Strom in der Wasserelektrolyseanlage zur Erzeugung von Wasserstoff- und Sauerstoffgas herangezogen werden. Während der Wasserstoff unmittelbar zur Anreicherung des Synthesegases der Methanolsyntheseanlage heranziehbar ist, lässt sich der gleichzeitig erzeugte Sauerstoff dem Kohlevergaser zuleiten. Er substituiert dort einen Teil des sonst von der Luftzerlegungsanlage gelieferten Sauerstoffs, so dass die Luftzerlegungsanlage in ihrer Leistung zurückgefahren und somit Energie eingespart werden kann.

Weitere Einzelheiten der Erfindung werden anhand von zwei in den Figuren dargestellten Ausführungsbeispielen erläutert:

Fig. 1 ein Mittellastkraftwerk mit einer integrierten Kohlevergasungsanlage und einer der Methanolsyntheseanlage zugeordneten Wasserelektrolyseanlage in schematischer Darstellung,

Fig. 2 ein anderes Mittellastkraftwerk mit einer integrierten Kohlevergasungsanlage und einem der Methanolsyntheseanlage zugeordneten sogenannten «Kühler-Sättiger-Kreislauf» in schematischer Darstellung und

Fig. 3 eine Variante für den Anschluss der Methanolsyntheseanlage der Fig. 2 an den sogenannten «Kühler-Sättiger-Kreislauf».

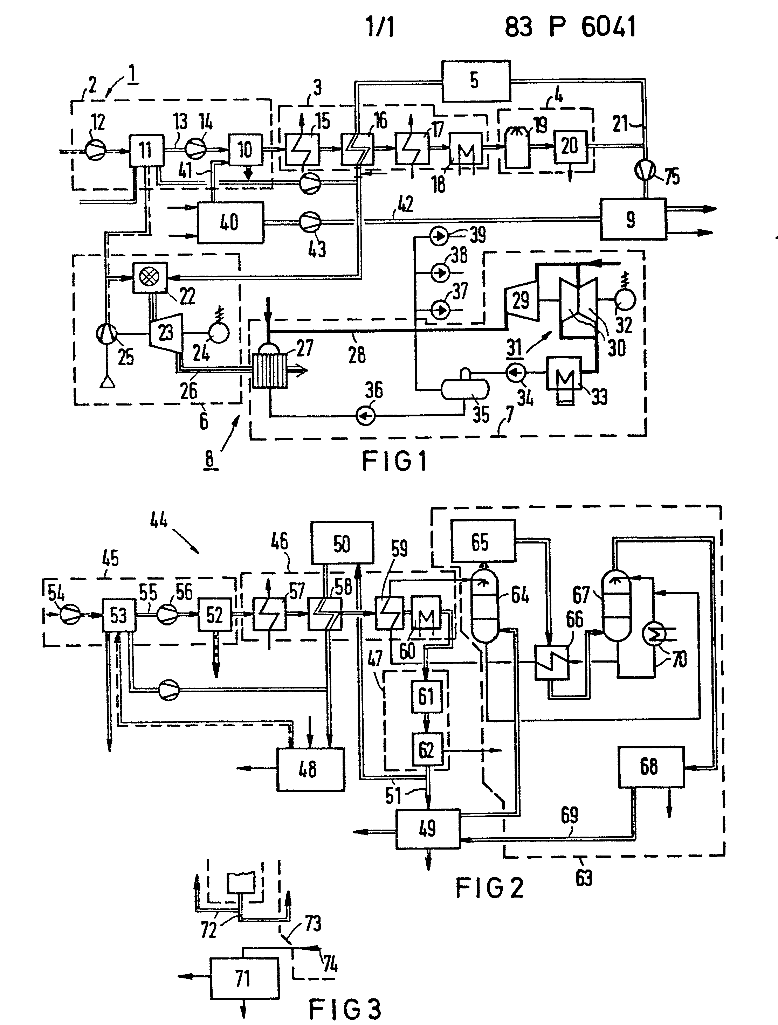

In der Darstellung der Fig. 1 sind die übergeordneten Baugruppen des Mittellastkraftwerks 1 gestrichelt umrandet. Es sind dies eine Kohlevergasungsanlage 2, eine Rohgas-Wärmetauscheranlage 3, eine Gasreinigungsanlage 4, ein zentrales Reingasverteilungssystem 5 mit einer integrierten Druckhaltungs- und Speicheranlage (der Übersichtlichkeit halber nicht dargestellt), ein aus einem Gasturbinenkraftwerksteil 6 und einem Dampfkraftwerksteil 7 bestehendes Kombikraftwerk 8 und eine Methanolsyntheseanlage 9. Die Kohlevergasungsanlage 2 beinhaltet einen Kohlevergaser 10, eine Luftzerlegungsanlage 11, mindestens einen der Luftzerlegungsanlage vorgeschalteten Zusatzluftverdichter 12 und einen in der von der Luftzerlegungsanlage 11 zum Kohlevergaser 10 führenden Sauerstoffleitung 13 angeordneten weiteren Gasverdichter 14. Die im Gasstrom des Kohlevergasers 10 angeordnete Rohgas-Wärmetauscheranlage 3 beinhaltet einen ersten, der Erzeugung von Hochdruckdampf dienenden Wärmetauscher 15, einen zweiten Rohgas-Reingas-Wärmetauscher 16 und einen dritten, der Erzeugung von Niederdruckdampf dienenden Wärmetauscher 17. Schliesslich ist in der Rohgas-Wärmetauscheranlage 3 noch ein Regelkühler 18 vorgesehen. Die der Rohgas-Wärmetauscheranlage nachgeschaltete Gasreinigungsanlage 4 beinhaltet einen Rohgaswäscher 19 sowie eine Schwefelwasserstoffabsorptions- und Schwefelgewinnungsanlage 20. An der die Schwefelwasserstoffabsorptions- und Schwefelgewinnungsanlage 20 verlassenden Reingasleitung 21 ist das Reingasverteilungssystem 5, die Methanolsyntheseanlage 9 und über den Rohgas-Reingas-Wärmetauscher 16 der Gasturbinenkraftwerksteil 6 angeschlossen.

Der Gasturbinen-Kraftwerksteil umfasst eine Brennkammer 22, eine Gasturbine 23 und je einen von der Gasturbine angetriebenen Generator 24 und Luftverdichter 25.

Die Abgasleitung 26 der Gasturbine 23 ist an einen Abhitzekessel 27 angeschlossen. Dessen Dampfleitung 28 ist mit dem Hochdruckteil 29 einer aus einem Hochdruckteil und Niederdruckteil 30 bestehenden Dampfturbine 31 verbunden. Mit der Dampfturbine 31 ist ein Generator 32 gekuppelt. Dem Niederdruckteil 30 der Dampfturbine 31 sind ein Kondensator 33, eine Kondensatpumpe 34, ein Speisewasserbehälter 35 sowie mehrere Speisewasserpumpen 36, 37, 38, 39 nachgeschaltet. An dem von der Gasturbine 23 angetriebenen Luftverdichter 25 ist sowohl die Brennkammer 22 der Gasturbine als auch die Luftzerlegungsanlage 11 der Kohlevergasungsanlage 2 angeschlossen. Der Kohlevergasungsanlage ist eine Wasserelektrolyseanlage 40 zugeordnet. Deren Sauerstoffleitung 41 ist parallel zur Sauerstoffleitung 13 der Luftzerlegungsanlage 11 an den Kohlevergaser 10 angeschlossen. Die Wasserstoffleitung 42 der Wasserelektrolyseanlage 40 ist über einen Gasverdichter 43 mit der Methanolsyntheseanlage 9 verbunden.

Beim Betrieb des Mittellastkraftwerks 1 wird die Luftzerlegungsanlage 11 sowohl durch den von der Gasturbine 23 angetriebenen Luftverdichter 25 als auch den Zusatz-Luftverdichter 12 mit Luft versorgt. Der Sauerstoff der Luftzerlegungsanlage wird über den Gasverdichter 14 in den Kohlevergaser 10 gedrückt. Im Kohlevergaser wird Kohle mit Sauerstoff und eingeleitetem Prozessdampf zu Rohgas vergast. Das 800 bis 1600° heisse Rohgas gibt seine Wärme in der Wärmetauscheranlage 3 ab, wobei im ersten Wärmetauscher 15 Hochdruckdampf erzeugt wird. Im zweiten Rohgas-Reingas-Wärmetauscher 16 wird das zur Brennkammer 22 des Gasturbinen-Kraftwerkteils 6 strömende Reingas vorgewärmt. Im dritten Wärmetauscher 17 wird Niederdruckdampf erzeugt, der dem Niederdruckteil 30 der Dampfturbine 31 zuleitbar oder auch als Prozessdampf verwertbar ist. Der Regelkühler 18 dient der definierten Temperierung des Rohgases vor Eintritt in den Rohgaswäscher 19. Die Druckhaltung, in der die Gasreinigungsanlage 4 verlassenen Reingasleitung 12 erfolgt, über das Reingas-Verteilungssystem 5 mit einer integrierten Reingas-Durchström-Zwischenspeicheranlage.

Die Methanolsyntheseanlage 9, die in mehrere separat zuschaltbare Module unterteilt ist, bleibt beim Betrieb des Mittellastkraftwerks 1 mit Nennlast zumindest mit einem Modul, das im Durchlaufbetrieb arbeitet, eingeschaltet. In sogenannten Schwachlastzeiten, wenn weniger elektrische Leistung an das Netz abgegeben wird, wird zunächst der Gasturbinenkraftwerksteil 6 zurückgefahren. Der Überschuss an Reingas wird durch Hochfahren der jeweils gerade in Betrieb befindlichen Module der Methanolsyntheseanlage 9 bzw. durch Zuschalten weiterer Module aufgefangen. So kann die Kohlevergasungsanlage 2 im optimalen Lastbereich weiter betrieben werden. Zugleich lässt sich die Wasserelektrolyseanlage mit einem Teil des überschüssig produzierten Stroms bei gleichzeitig zurückgenommener Leistung des Gasturbinenkraftwerksteils in Betrieb nehmen. Der dabei erzeugte Wasserstoff lässt sich über den Verdichter 43 in die Methanolsyntheseanlage 9 einspeisen. Hierdurch wird die Zusammensetzung des in der Methanolsyntheseanlage eingespeisten Reingases bzw. des in der Methanolsyntheseanlage rezirkulierenden Synthesegases an das für die Methanolsynthese erforderliche stöchiometrische Verhältnis herangebracht. Der zugleich bei der Wasserelektrolyse anfallende Sauerstoff wird in den Kohlevergaser 10 eingeleitet. Er substituiert dort einen Teil des Sauerstoffs aus der Luftzerlegungsanlage 11. Das hat zur Folge, dass die Luftzerlegungsanlage 11 in ihrer Leistung zurückgenommen werden kann. Bei dieser Lösung kann die in Zeiten verminderten Strombedarfs erzeugte Methanolmenge durch Anpassung der Synthesegaszusammensetzung an das stöchiometrische Verhältnis durch den mit überschüssiger elektrischer Energie erzeugtem Wasserstoff so gesteigert werden, dass die gesamte bei Nennlast des Kohlevergasers 10 erzeugte Reingasmenge, die nicht vom Gasturbinenkraftwerksteil 6 benötigt wird, bis auf die inerten Gasbestandteile vollständig in Methanol umgewandelt wird.

Eine weitere Erhöhung der erzeugten Methanolmenge wird erreicht, wenn zusätzlich kohlenwasserstoffhaltiges Gas aus einer externen Quelle (nicht dargestellt) zu Synthesegas gespalten und dieses Gas in die Methanolsyntheseanlage eingespeist wird. In diesem Fall lässt sich sogar beim Extremfall einer vollständigen Abtrennung des Mittellastkraftwerks 1 vom elektrischen Netz, dessen volle elektrische Leistung in die Wasserelektrolyseanlage 40 einspeisen. Weil bei dieser Betriebsweise des Mittellastkraftwerks nur eine geringe Menge des vom Kohlevergaser erzeugten Reingases für die Methanolsynthese verfügbar ist, steht dieser Wasserstoff nahezu vollständig für die Methanolsynthese aus dem aus der externen Quelle eingespeisten kohlenwasserstoffhaltigen Gas zur Verfügung. So kann bei allen denkbaren Lastfällen unabhängig davon, ob das aus Gasturbinenteil 6 und Dampfkraftwerksteil 7 bestehende Kombikraftwerk 8 in Zeiten verminderten Strombedarfs mit Nennlast weiter betrieben wird oder ob dessen Leistung in solchen Zeiten zurückgenommen wird, die Kohlevergasungsanlage 2 mit Nennlast weiter betrieben und das überschüssig erzeugte Reingas oder/und gleichzeitig aus der Spaltung zusätzlichen kohlenwasserstoffhaltigen Gases gebildetes Synthesegas in Methanol umgewandelt werden.

Auch das Mittellastkraftwerk 44 des in Fig. 2 gezeigten Ausführungsbeispiels besteht aus einer Kohlevergasungsanlage 45, einer Rohgaswärmetauscheranlage 46, einer Gasreinigungsanlage 47, aus einem Gasturbinen-Kraftwerksteil und einem Dampfkraftwerksteil bestehenden Kombikraftwerk 48, einer Methanolsyntheseanlage 49 und einem zentralen Reingas-Verteilungssystem 50 mit einer parallel zur Reingasleitung 51 geschalteten Reingas-Durchströmzwischenspeicheranlage (der Übersichtlichkeit halber nicht dargestellt). Auch hier beinhaltet die Kohlevergasungsanlage 45 einen Kohlevergaser 52, eine Luftzerlegungsanlage 53, einen der Luftzerlegungsanlage vorgeschalteten Zusatzluftverdichter 54 und einen in der Sauerstoffleitung 55 zum Kohlevergaser angeordneten weiteren Gasverdichter 56. Auch die dem Gasstrom des Kohlevergasers 52 zugeordnete Rohgas-Wärmetauscheranlage 46 beinhaltet einen der Dampferzeugung dienenden Wärmetauscher 57, einen Rohgas-Reingaswärmetauscher 58, einen ebenfalls der Heisswassererzeugung dienenden Wärmetauscher 59 und einen Regelkühler 60. Auch die der Rohgas-Wärmetauscheranlage 46 nachgeschaltete Gasreinigungsanlage 47 beinhaltet einen Rohgaswäscher 61 und eine Schwefelwasserstoffabsorbtions- und Schwefelgewinnungsanlage 62.

An der die Gasreinigungsanlage verlassenden Reingasleitung 51 ist, ähnlich wie im Ausführungsbeispiel der Figur 1, sowohl das zentrale Reingasverteilungssystem 50, die Methanolsyntheseanlage 49 und über den Reingas-Rohgas-Wärmetauscher 58 das Kombikraftwerk 48 angeschlossen. Letzteres ist genauso aufgebaut, wie das anhand des Ausführungsbeispiels der Figur 1 im einzelnen dargestellt worden ist.

Abweichend vom Ausführungsbeispiel der Figur 1 ist an der Methanolsyntheseanlage 49 ein sogenannter «Kühler-Sättiger-Kreislauf» 63 angeschlossen. Dieser besteht aus einem Sättiger 64, einer Konvertierungsanlage 65, einem Wärmetauscher 66, einem Kühler 67 und einer Gasreinigungsanlage 68. Das im Kühler-Sättigungs-Kreislauf mit Wasserstoff angereicherte Synthesegas wird über eine Rezirkulationsleitung 69 in die Methanolsyntheseanlage 49 zurückgeführt und in die jeweils in Betrieb befindlichen Synthese-Reaktoren (der Übersichtlichkeit halber nicht dargestellt) der Methanolsyntheseanlage eingeleitet.

Beim Betrieb des Mittellastkraftwerkes 44 wird in ähnlicher Weise, wie dies anhand des Ausführungsbeispiels der Fig. 1 bereits beschrieben worden ist, im Kohlevergaser 52 mit dem Sauerstoff der Luftzerlegungsanlage 53 und mit Wasserdampf Rohgas erzeugt. Dieses Rohgas wird in der nachgeschalteten Rohgas-Wärmetauscheranlage 46 abgekühlt und in der Gasreinigungsanlage 47 gereinigt. Mit dem so hergestellten Reingas wird über das Reingasverteilungssystem 50, den Rohgas-Reingas-Wärmetauscher 58 das aus einem Gasturbinenkraftwerksteil und einem Dampfkraftwerksteil bestehende Kombikraftwerk 48 betrieben. Dabei wird auch der im ersten Wärmetauscher 57 der Rohgas-Wärmetauscheranlage 46 erzeugte Hochdruckdampf in die Dampfturbine des Dampfkraftwerksteils eingespeist. Das in den gerade in Betrieb befindlichen Modulen der Methanolsyntheseanlage 49 teilweise umgesetzte Synthesegas wird in den Sättiger 64 geleitet und dort mittels heissem Wasser, welches dem dritten Wärmetauscher 59 der Rohgas-Wärmetauscheranlage 46 entnommen wird, mit Wasserdampf gesättigt. Das so erhaltene Mischgas wird in der nachgeschalteten Konvertierungsanlage 65 umgewandelt, wobei das Kohlenmonoxyd bei gleichzeitiger Spaltung des Wassers zu Kohlendioxyd aufoxidiert wird. Das Abgas der Konvertierungsanlage 65 wird in einem ersten Wärmetauscher 66 gekühlt, wobei das in diesem Wärmetauscher aufgewärmte Kühlwasser zur weiteren Aufheizung in den dritten Wärmetauscher 59 der Rohgas-Wärmetauscheranlage 46 eingespeist wird. Das so vorgekühlte Abgas der Konvertierungsanlage 65 wird in einem weiteren, an einen Kühlerkreislauf 70 angeschlossenen Kühler 67 nachgekühlt und in die Gasreinigungsanlage 68 eingeleitet. In dieser Gasreinigungsanlage wird das Kohlendioxyd ausgewaschen und das verbleibende mit Wasserstoff angereicherte Gas als Synthesegas über die Rezirkulationsleitung 69 wieder in die Methanolsyntheseanlage 49 zurückgeleitet. Hier wird es in einen der in Betrieb befindlichen Synthesereaktoren eingespeist.

Es wäre auch möglich, aus dem Abgas der Konvertierungsanlage in einer Gaszerlegungsanlage eine wasserstoffreiche Fraktion zu gewinnen. Ferner wäre es möglich, anstelle des den Synthesereaktoren der Methanolsyntheseanlage entströmende Synthesegases das erstmals in die Methanolsyntheseanlage einströmende Reingas über den Kühler-Sättigerkreislauf mit Wasserstoff anzureichern, so dass das stöchiometrische Verhältnis für die Methanolerzeugung erreicht wird.

Dieses mit Wasserstoff angereicherte Synthesegas könnte dann in die Methanolsyntheseanlage eingespeist werden und dort so lange durch die einzelnen Synthesereaktoren rezirkulieren, bis es vollständig, d.h. bis auf die Inertgasreste zu Methanol umgesetzt ist. Die Schaltung der Methanolsyntheseanlage 71 für diese Art der Vorweganreicherung des Reingases mit Wasserstoff ist im Ausführungsbeispiel der Fig. 3 dargestellt. Man erkennt hier, dass die Reingasleitung 72 zunächst in den sonst unveränderten Kühler-Sättiger-Kreislauf 73 eingespeist wird und erst das angereicherte und von Kohlendioxyd befreite Abgas der Konvertierungsanlage hinter der Gasreinigungsanlage über die Rezirkulationsleitung 74 in die Methanolsyntheseanlage 71 eingeleitet wird.

Auch kann die Gasturbine zu Zeiten, in denen weniger Energie ins elektrische Netz eingespeist wird, heruntergefahren bzw. abgeschaltet werden und das nunmehr vermehrt verfügbare Reingas über die Methanolsyntheseanlage 49, 71 unter Anreicherung des Synthesegases mit Wasserstoff zu Methanol umgeformt werden. Dabei kann die nunmehr im dritten Wärmetauscher 58 der Rohgas-Wärmetauscheranlage 46 wegen des abgeschalteten Rohgas-Reingaswärmetauschers 58 vermehrt anfallende Wärme zur weiteren Aufsättigung des Reingases und unter Umständen zur zusäztlichen Spaltung von extern eingeführten kohlenwasserstoffhaltigem Gas genutzt werden. Durch die Erhöhung des Synthesegaserzeugung kann mehr Methanol erzeugt werden.

Bezugszeichenliste

| | | |
|---|---|---|
| Mittellastkraftwerk | 1 | 44 |
| Kohlevergasungsanlage | 2 | 45 |
| Rohgas-Wärmetauscheranlage | 3 | 46 |
| Gasreinigungsanlage | 4 | 47 |
| Reingasverteilungssystem | 5 | 50 |
| Gasturbinenkraftwerksteil | 6 | |
| Dampfkraftwerksteil | 7 | |
| Kombikraftwerk | 8 | 48 |
| Methanolsyntheseanlage | 9 | 49, 71 |
| Kohlevergaser | 10 | 52 |
| Luftzerlegungsanlage | 11 | 53 |
| Zusatzluftverdichter | 12 | 54 |
| Sauerstoffleitung | 13 | 55 |
| Gasverdichter | 14 | 56 |
| Wärmetauscher | 15 | 57 |
| Rohgas-Reingas-Wärmetauscher | 16 | 58 |
| Wärmetauscher | 17 | 59 |
| Regelkühler | 18 | 60 |
| Rohgaswäscher | 19 | 61 |
| Schwefelwasserstoffabsorptions- und Schwefelgewinnungsanlage | 20 | 62 |
| Reingasleitung | 21 | 51, 72 |
| Brennkammer | 22 | |
| Gasturbine | 23 | |
| Generator | 24 | |
| Luftverdichter | 25 | |
| Abgasleitung | 26 | |
| Abhitzekessel | 27 | |
| Dampfleitung | 28 | |
| Hochdruckteil | 29 | |
| Niederdruckteil | 30 | |
| Dampfturbine | 31 | |
| Generator | 32 | |
| Kondensator | 33 | |
| Kondensatpumpe | 34 | |
| Speisewasserbehälter | 35 | |
| Speisewasserpumpe | 36, 37, 38, 39 | |
| Wasserelektrolyseanlage | 40 | |
| Sauerstoffleitung | 41 | |
| Wasserstoffleitung | 42 | |
| Gasverdichter | 43 | |
| Kühler-Sättiger-Kreislauf | 63, 73 | |
| Sättiger | 64 | |
| Konvertierungsanlage | 65 | |
| Wärmetauscher | 66 | |
| Kühler | 67 | |
| Gasreinigungsanlage | 68 | |
| Rezirkulationsleitung | 69, 74 | |
| Kühlerkreislauf | 70 | |

**Patentansprüche**

1. Mittellastkraftwerk (44) mit einer integrierten Kohlevergasungsanlage (45) mit einem an die Kohlevergasungsanlage angeschlossenen Gasturbinenkraftwerksteil (48), mit einem an die Rohgas-Wärmetauscheranlage (46) der Kohlevergasungsanlage angeschlossenen Dampfkraftwerksteil (48) mit einer aus mehreren untereinander parallelgeschalteten Modulen bestehenden Methanolsyntheseanlage (49, 71) und mit einem die Methanolsyntheseanlage mit dem Gasturbinenkraftwerksteil verbindenden Reingasverteilungssystem (50), welches eine Reingasdurchström-Zwischenspeicheranlage umfasst und gasseitig der Rohgas-Wärmetauscheranlage nachgeschaltet ist, dadurch gekennzeichnet, dass zur stärkeren Annäherung des Verhältnisses von CO zu $H_2$ an das bei der Methanolsynthese erforderliche stöchiometrische Verhältnis, der Methanolsyntheseanlage (49, 71) ein aus Sättiger (64), Konvertierungsanlage (65), Kühler (66, 67) und nachgeschalteter Gasreinigungsanlage (68) bestehender sogenannter «Kühler-Sättiger-Kreislauf» (63, 73) zugeordnet ist, in welchem Sättiger Heisswasser aus einem Wärmetauscher (59) der Rohgas-Wärmetauscheranlage (46) eingespeist wird und welches Wasser darin bis zur Sättigung des Gases verdampft.

2. Mittellastkraftwerk (44) mit einer integrierten Kohlevergasungsanlage (2), mit einem an die Kohlevergasungsanlage angeschlossenen Gasturbinenkraftwerksteil (6), mit einem an die Rohgas-Wärmetauscheranlage (3) der Kohlevergasungsanlage angeschlossenen Dampfkraftwerksteil (7), mit einer aus mehreren untereinander parallelgeschalteten Modulen bestehenden Methanolsyntheseanlage (9) und mit einem die Methanolsyntheseanlage (9) mit dem Gasturbinenkraftwerksteil (6) verbindenden Reingasverteilungssystem (5), welches eine Reingasdurchström-Zwischenspeicheranlage umfasst und gasseitig der Rohgas-Wärmetauscheranlage (46) nachgeschaltet ist, dadurch gekennzeichnet, dass der Methanolsyntheseanlage (9) eine Wasserelektrolyseanlage (4) zugeordnet ist, deren Wasserstoffleitung (42) mit der Methanolsyntheseanlage verbunden, deren Sauerstoffleitung (41) an den Kohlevergaser (10) angeschlossen ist, und die über die Generatoren (24, 32) mit im wesentlichen der Menge an elektrischer Energie versorgt werden kann, die der Differenz zwischen Nennleistung und momentaner Last entspricht.

3. Mittellastkraftwerk nach Anspruch 2, dadurch gekennzeichnet, dass der Kohlevergaser (10) mit dem gleichen Druck wie die Wasserelektrolyseanlage (40) arbeitet und je ein Verdichter (43, 75) in der Reingaszuleitung (21) und in der Wasserstoffzuleitung (42) zur Methanolsyntheseanlage eingebaut ist.

4. Mittellastkraftwerk nach Anspruch 2, dadurch gekennzeichnet, dass der Kohlevergaser (10) mit dem gleichen Druck wie der Methanolsynthesereaktor (9) arbeitet und in der Sauerstoffleitung (41) zwischen der Wasserelektrolyseanlage (40) und dem Kohlevergaser sowie in der Wasserstoffleitung (42) zum Methanolsynthesereaktor je ein Verdichter (43) eingebaut ist.

5. Mittellastkraftwerk nach Anspruch 2, dadurch gekennzeichnet, dass die Wasserstoffleitung (42) mit der zum Synthesereaktor der Methanolsyntheseanlage (9) führenden Reingasleitung (21) verbunden ist.

6. Mittellastkraftwerk nach Anspruch 2, dadurch gekennzeichnet, dass die Wasserstoffleitung mit der zum Synthesereaktor der Methanolsyntheseanlage führenden Leitung für das rezirkulierende Synthesegas verbunden ist.

7. Mittellastkraftwerk nach Anspruch 1, dadurch gekennzeichnet, dass der «Kühler-Sättiger-Kreislauf» (63) in der in den Synthesereaktor der Methanolsyntheseanlage (49) zurückführenden Synthesegasleitung (69) eingeschaltet ist.

8. Mittellastkraftwerk nach Anspruch 1, dadurch gekennzeichnet, dass der «Kühler-Sättiger-Kreislauf» (73) in der zum Synthesegasreaktor der Methanolsyntheseanlage (71) führenden Leitung (72) für das Reingas eingeschaltet ist.

9. Mittellastkraftwerk nach Anspruch 1, dadurch gekennzeichnet, dass der Sättiger (64) über eine separate, mit einem Drosselventil versehene Leitung aus einem Wärmetauscher (57) der Rohgas-Wärmetauscheranlage (46) mit Hochdruckdampf speisbar ist.

10. Mittellastkraftwerk nach Anspruch 1, dadurch gekennzeichnet, dass die Gasreinigungsanlage (68) eine Kohlendioxidwaschanlage beinhaltet.

11. Mittellastkraftwerk nach Anspruch 1, dadurch gekennzeichnet, dass die Gasreinigungsanlage eine Wasserstoffabtrennanlage beinhaltet.

**Revendications**

1. Centrale à charge moyenne (44), avec une installation intégrée de gazéification du charbon (45), une partie de centrale à turbine à gaz (48), reliée à l'installation de gazéification du charbon, une partie de centrale à vapeur (48) reliée à l'installation à èchangeurs de chaleur pour le gaz brut (46) de l'installation de gazéification du charbon, une installation de synthèse du méthanol (49, 71), constituée de plusieurs modules montés en parallèle les uns aux autres et un système de répartition du gaz purifié (50), qui relie l'installation de synthèse du méthanol avec la partie de centrale à turbine à gaz, qui comprend une installation de stockage intermédiaire à écoulement continu du gaz purifié et qui est monté en aval du côté du gaz de l'installation à échangeurs de chaleur pour le gaz purifié, caractérisée en ce que pour le rapport de CO à $H_2$ soit très proche du rapport stoechiométrique nécessaire pour la synthèse du méthanol, il est associé à l'installation de synthèse du méthanol (49, 71) ce que l'on appelle un «circuit refroidisseur-saturateur» (63, 73) qui est constitué d'un saturateur (64), d'une installation de conversion (65), d'un refroidisseur (66, 67) et d'une installation de purification du gaz (68) montée en aval, de l'eau chaude provenant d'un échangeur (59) de l'installation à échangeurs de chaleur pour le gaz brut (46) étant envoyée dans le saturateur et cette eau chaude y étant évaporée jusqu'à la saturation du gaz.

2. Centrale à charge moyenne (44), comprenant une installation intégrée de gazéification du charbon (2), une partie de centrale à turbine à gaz (6) reliée à l'installation de gazéification du charbon, une partie de centrale à vapeur (7) reliée à l'installation à échangeurs de chaleur pour le gaz brut (3) de l'installation de gazéification du charbon, une installation de synthèse du méthanol (9) constituée de plusieurs modules montés en parallèle les uns aux autres et un système de répartition du gaz purifié (5), mettant en communication l'installation de synthèse du méthanol (9) et la partie de centrale à turbine à gaz (6), comprenant une installation de stockage intermédiaire à écoulement continu du gaz purifié et monté, en aval suivant la direction du gaz, de l'installation à échangeurs de chaleur pour le gaz brut, caractérisée en ce qu'à l'installation de synthèse du méthanol (9) est associée une installation d'électrolyse de l'eau (4), dont le conduit pour l'hydrogène (42) communique avec l'installation de synthèse du méthanol, dont le conduit pour l'oxygène (41) communique avec le gazéificateur de charbon (10) et qui peut être alimentée par des génératrices (24, 32) en la quantité d'énergie électrique qui correspond sensiblement à la différence entre la puissance nominale et la puissance instantanée.

3. Centrale à charge monenne suivant la revendication 2, caractérisée en ce que le gazéificateur de charbon (10) fonctionne sous la même pression que l'installation d'électrolyse de l'eau (40) et des compresseurs (43, 75) sont montés respectivement dans le conduit d'amenée du gaz purifié (21) et dans le conduit d'amenée de l'hydrogène (42), allant à l'installation de synthèse du méthanol.

4. Centrale à charge moyenne suivant la revendication 2, caractérisée en ce que le gazéificateur du charbon (10) fonctionne sous la même pression que le réacteur de synthèse du méthanol et des compresseurs (43) sont montés respectivement dans le conduit pour l'oxygène (41) entre l'installation d'électrolyse de l'eau (40) et le gazéificateur du charbon, ainsi que dans le conduit pour l'hydrogène (42) allant au réacteur de synthèse du méthanol.

5. Centrale à charge moyenne suivant la revendication 2, caractérisée en ce que le conduit pour l'hydrogène (42) communique avec le conduit (21) pour le gaz purifié menant au réacteur de synthèse de l'installation de synthèse du méthanol (9).

6. Centrale à charge moyenne suivant la revendication 2, caractérisée en ce que le conduit pour l'hydrogène communique avec le conduit pour le gaz de synthèse recyclè menant au réacteur de synthèse de l'installation de synthèse du méthanol.

7. Centrale à charge moyenne suivant la revendication 1, caractérisée en ce que le «circuit refroidisseur-saturateur» (63) est monté dans le conduit pour le gaz de synthèse (69) retournant au réacteur de synthèse de l'installation de synthèse du méthanol (49).

8. Centrale à charge moyenne suivant la revendication 1, caractérisée en ce que le «circuit refroidisseur-saturateur» (75) est monté dans le conduit (72) pour le gaz purifié allant au réacteur de synthèse de l'installation de synthèse du méthanol (71).

9. Centrale à charge moyenne suivant la revendication 1, caractérisée en ce que le saturateur (64) peut être alimenté en vapeur à haute pression par un conduit distinct muni d'une vanne d'étranglement et provenant d'un échangeur de chaleur (57) de l'installation à échangeurs de chaleur pour le gaz brut (46).

10. Centrale à charge moyenne suivant la revendication 1, caractérisée en ce que l'installation de purification du gaz (68) comporte une installation d'élimination du dioxyde de carbone par lavage.

11. Centrale à charge moyenne suivant la revendication 1, caractérisée en ce que l'installation de purification du gaz comporte une installation de séparation de l'hydrogène.

**Claims**

1. Medium load power station (44) with an integrated coal gasification plant (45), with a gas turbine power station section (48) connected to the coal gasification plant, with a steam power station section (48) connected to the crude gas heat exchanger plant (46) of the coal gasification plant, with a methanol synthesis plant (49, 71) consisting of several modules connected in parallel to one another, and with a pure gas distribution system (50) linking the methanol synthesis plant with the gas turbine power station section, which system comprises a pure gas-throughflow-intermediate storage plant and is connected downstream on the gas-side of the crude gas heat exchanger plant, characterised in that for a greater approximation of the ratico CO to $H_2$ to the stochiometric ratio required with the methanol synthesis, there is coordinated to the methanol synthesis plant (49, 71) a so-called «cooler-saturator-circulation system» (63, 73) consisting of saturator (64), converting plant (65), cooler (66, 67) and downstream gas purification plant (68), in which saturator hot water is fed from a heat exchanger (59) of the crude gas heat exchanger plant (46) and which water vaporizes in it until saturation of the gas.

2. Medium load power station (44) with an integrated coal gasification plant (2) with a gas turbine power station section (6) connected to the coal gasification plant, with a steam power station section (7) connected to the crude gas heat exchanger plant (3) of the coal gasification plant, with a methanol synthesis plant (9) consisting of several modules connected in parallel to one another, and with a pure gas distribution system (5) linking the methanol synthesis plant (9) with the gas turbine power station section (6), which system comprises a pure gas-throughflow-intermediate storage plant and is connected downstream on the gas-side of the crude gas heat exchanger plant (46), characterised in that to the methanol synthesis plant (9) there is coordinated a water electrolysis plant (4), the hydrogen line (42) of which is linked to the methanol synthesis plant, the oxygen line (41) of which is connected to the coal gasifier (10) and which by way of the generators (24, 32) can be supplied with essentially the amount of electric energy, which corresponds to the difference between nominal output and momentary load.

3. Medium load power station in accordance with claim 2, characterised in that the coal gasifier (10) works at the same pressure as the water electrolysis plant (40) and that a compressor (43, 75) is built respectively into the pure gas line (21) and the hydrogen line (42) leading to the methanol synthesis plant.

4. Medium load power station in accordance with claim 2, characterised in that the coal gasifier (10) works at the same pressure as the methanol synthesis reactor (9) and that a compressor (43) is built respectively into the oxygen line (41) between the water electrolysis plant (40) and the coal gasifier and into the hydrogen line (42) to the methanol synthesis reactor.

5. Medium load power station in accordance with claim 2, characterised in that the hydrogen line (42) is linked to the pure gas line (21) leading to the synthesis reactor of the methanol synthesis plant (9).

6. Medium load power station in accordance with claim 2, characterised in that the hydrogen line is linked to the line, leading to the synthesis reactor of the methanol synthesis plant, for the recirculating synthesis gas.

7. Medium load power station in accordance with claim 1, characterised in that the «cooler-saturator-circulation system» (63) is arranged in the synthesis gas line (69) leading back into the synthesis reactor of the methanol synthesis plant (49).

8. Medium load power station in accordance with claim 1, characterised in that the «cooler-saturator-circulation system» (73) is arranged in the line (72) for the pure gas leading to the synthesis gas reactor of the methanol synthesis plant (71).

9. Medium load power station in accordance with claim 1, characterised in that the saturator (64) can be fed with high pressure steam by way of a separate line, provided with a throttle valve, from a heat exchanger (57) of the crude gas heat exchanger plant (46).

10. Medium load power station in accordance with claim 1, characterised in that the gas purification plant (68) contains a carbon dioxide washing plant.

11. Medium load power station in accordance with claim 1, characterised in that the gas purification plant contains a hydrogen separation plant.

FIG 1

FIG 2

FIG 3